# EUROPEAN PATENT APPLICATION

(11) **EP 4 368 636 A1**
(43) Date of publication of application: **15.05.2024**
(21) Application number: 22836884.1
(22) Date of filing: 05.07.2022
(51) Int. Cl.: C07K 14/605, C07K 19/00, A61K 38/26, A61K 47/68

(54) **FUSION PROTEIN AND APPLICATION THEREOF**

(30) Priority: 06.07.2021 CN 202110764902; 24.06.2022 CN 202210721402
(71) Applicant: Suzhou Alphamab Co., Ltd., Jiangsu 215125 (CN)
(72) Inventor: XU, Ting, Suzhou, Jiangsu 215125 (CN); JIN, Yuhao, Suzhou, Jiangsu 215125 (CN); WANG, Xiaoxiao, Suzhou, Jiangsu 215125 (CN); WANG, Pilin, Suzhou, Jiangsu 215125 (CN); GUO, Kangping, Suzhou, Jiangsu 215125 (CN)
(74) Representative: Henderson, Helen Lee
(86) International application number: PCT/CN2022/103777
(87) International publication number: WO 2023/280133

(57) **Abstract**

The present application relates to a fusion protein and use thereof. The fusion protein comprises a human GLP-1 polypeptide variant and an immunoglobulin Fc region, wherein an amino acid sequence of the human GLP-1 polypeptide variant comprises at least 2 amino acid mutations compared with an amino acid sequence set forth in SEQ ID NO: 2, and the at least 2 amino acid mutations are located at amino acid positions selected from the group consisting of W31, K26, and Y19. The present application also provides use of the fusion protein in treating a disease or a condition.

## Description

### TECHNICAL FIELD

The present application relates to the field of biomedicine and in particular to a fusion protein of a GLP-1 polypeptide variant and an immunoglobulin Fc region and use thereof.

### BACKGROUND

Glucagon-like peptide-1 (GLP-1) is an incretin secreted by L-cells of the small intestinal epithelium. In healthy individuals, GLP-1 is capable of promoting insulin release and inhibiting glucagon secretion in response to the uptake of nutrients by the body. In patients with type II diabetes, administration of a supraphysiological dose of GLP-1 can restore endogenous insulin secretion and lower blood sugar.

The plasma half-life of native GLP-1 is short due to the enzymolysis by dipeptidyl peptidase IV (DPPIV) and clearing in kidney. Although several GLP-1R agonists modified to prolong the half-life (e.g., dulaglutide) have been developed for the treatment of type II diabetes, there is still a need to develop an effective long-term treatment regimen while maintaining minimal side effects.

### SUMMARY

The present application provides a fusion protein comprising a human GLP-1 polypeptide variant and an immunoglobulin Fc region. The fusion protein has excellent pharmacokinetic properties, e.g., longer half-life (t1/2) or higher exposure. The fusion protein of the present application is not easy to be cleared and significantly improves the half-life of the native GLP-1, which is beneficial for enabling functioning of the GLP-1 polypeptide variant *in vivo,* improving the drug effect, and reducing the usage amount.

The present application provides a fusion protein. The fusion protein comprises a human GLP-1 polypeptide variant and an immunoglobulin Fc region, wherein an amino acid sequence of the human GLP-1 polypeptide variant comprises at least 2 amino acid mutations compared with an amino acid sequence set forth in SEQ ID NO: 2, and the at least 2 amino acid mutations are located at amino acid positions selected from the group consisting ofW31, K26, and Y19.

In certain embodiments, the immunoglobulin Fc region is located at the C-terminus of the human GLP-1 polypeptide variant.

In certain embodiments, the immunoglobulin Fc region is an Fc region derived from IgG.

In certain embodiments, the immunoglobulin Fc region is an Fc region derived from IgG1, IgG2, IgG3, or IgG4.

In certain embodiments, the immunoglobulin Fc region is an Fc region derived from IgG4.

In certain embodiments, the immunoglobulin Fc region comprises an amino acid sequence set forth in any one of SEQ ID NOs: 32-35.

In certain embodiments, the immunoglobulin Fc region comprises amino acid mutations, and the amino acid mutations selectively enhance binding affinity of the immunoglobulin Fc region for an Fc receptor compared with an immunoglobulin Fc region without amino acid mutations.

In certain embodiments, the immunoglobulin Fc region comprises amino acid mutations at positions M252, S254, and T256 according to EU numbering.

In certain embodiments, the immunoglobulin Fc region comprises amino acid mutations in the group consisting of M252Y, S254T, and/or T256E.

In certain embodiments, amino acid mutations of the immunoglobulin Fc region are M252Y, S254T, and T256E.

In certain embodiments, the immunoglobulin Fc region comprises an amino acid sequence set forth in SEQ ID NO: 36.

In certain embodiments, the fusion protein comprises a hinge region located between the human GLP-1 polypeptide variant and the immunoglobulin Fc region.

In certain embodiments, the hinge region is derived from IgG.

In certain embodiments, the hinge region is derived from IgG1 or IgG4.

In certain embodiments, the hinge region comprises an amino acid sequence set forth in any one of SEQ ID NOs: 30-31.

In certain embodiments, the hinge region is derived from IgG1.

In certain embodiments, the hinge region comprises an amino acid sequence set forth in SEQ ID NO: 30.

In certain embodiments, the fusion protein further comprises a linker located between the human GLP-1 polypeptide variant and the hinge region.

In certain embodiments, the linker is a peptide linker.

In certain embodiments, the linker has a length of 5-20 amino acids.

In certain embodiments, the linker has a length of 15 amino acids.

In certain embodiments, the linker comprises an amino acid sequence set forth in any one of SEQ ID NOs: 27-29.

In certain embodiments, the linker comprises an amino acid sequence set forth in SEQ ID NO: 28. In certain embodiments, the human GLP-1 polypeptide variant has at least partial activity of human GLP-1.

In certain embodiments, an amino acid sequence of the human GLP-1 polypeptide variant comprises at least 2 mutations at amino acid positions selected from the group consisting of W31, K26, and Y19 compared with the amino acid sequence set forth in SEQ ID NO: 2.

In certain embodiments, an amino acid sequence of the human GLP-1 polypeptide variant comprises mutations at amino acid positions W31 and K26 compared with the amino acid sequence set forth in SEQ ID NO: 2.

In certain embodiments, an amino acid sequence of the human GLP-1 polypeptide variant comprises mutations at amino acid positions W31 and Y19 compared with the amino acid sequence set forth in SEQ ID NO: 2.

In certain embodiments, an amino acid sequence of the human GLP-1 polypeptide variant comprises mutations at amino acid positions K26 and Y19 compared with the amino acid sequence set forth in SEQ ID NO: 2.

In certain embodiments, an amino acid sequence of the human GLP-1 polypeptide variant comprises mutations at amino acid positions W31, K26, and Y19 compared with the amino acid sequence set forth in SEQ ID NO: 2.

In certain embodiments, the human GLP-1 polypeptide variant comprises an amino acid substitution at the position W31, and the amino acid substitution is W31Y, W31R, W31K, or W31A.

In certain embodiments, the human GLP-1 polypeptide variant comprises an amino acid substitution at the position W31, and the amino acid substitution is W31Y.

In certain embodiments, the human GLP-1 polypeptide variant comprises an amino acid substitution at the position Y19, and the amino acid substitution is any amino acid substitution selected from the group consisting of Y19A, Y19L, Y19T, Y19F, Y19I, Y19V, and Y19S.

In certain embodiments, the human GLP-1 polypeptide variant comprises an amino acid substitution at the position Y19, and the amino acid substitution is any amino acid substitution selected from the group consisting of Y19A.

In certain embodiments, the human GLP-1 polypeptide variant comprises an amino acid substitution at the position K26, and the amino acid substitution is K26R.

In certain embodiments, the human GLP-1 polypeptide variant comprises an amino acid sequence set forth in SEQ ID NO: 26.

In certain embodiments, the human GLP-1 polypeptide variant comprises an amino acid sequence set forth in any one of SEQ ID NOs: 4-25.

In certain embodiments, an amino acid sequence of the human GLP-1 polypeptide variant comprises amino acid mutations W31Y, K26R, and Y19A compared with the amino acid sequence set forth in SEQ ID NO: 2.

In certain embodiments, the human GLP-1 polypeptide variant comprises an amino acid sequence set forth in SEQ ID NO: 25.

In certain embodiments, an amino acid sequence of the human GLP-1 polypeptide variant comprises amino acid mutations W31Y and K26R compared with the amino acid sequence set forth in SEQ ID NO: 2.

In certain embodiments, the human GLP-1 polypeptide variant comprises an amino acid sequence set forth in SEQ ID NO: 4.

In certain embodiments, the fusion protein comprises the human GLP-1 polypeptide variant and the immunoglobulin Fc region. In certain embodiments, the human GLP-1 polypeptide variant comprises an amino acid sequence set forth in SEQ ID NO: 4 or 25, and in certain embodiments, the immunoglobulin Fc region is an Fc region derived from IgG4. In certain embodiments, the Fc region comprises amino acid mutations M252Y, S254T, and T256E according to EU numbering. In certain embodiments, the Fc region comprises an amino acid sequence set forth in SEQ ID NO: 36.

In certain embodiments, the fusion protein comprises the human GLP-1 polypeptide variant and the hinge region. In certain embodiments, the human GLP-1 polypeptide variant comprises an amino acid sequence set forth in SEQ ID NO: 4 or 25. In certain embodiments, the hinge region is derived from IgG1. In certain embodiments, the hinge region comprises an amino acid sequence set forth in SEQ ID NO: 30.

In certain embodiments, the fusion protein comprises the human GLP-1 polypeptide variant, the immunoglobulin Fc region, and the hinge region. In certain embodiments, the human GLP-1 polypeptide variant comprises an amino acid sequence set forth in SEQ ID NO: 4 or 25. In certain embodiments, the immunoglobulin Fc region is an Fc region derived from IgG4. In certain embodiments, the hinge region is derived from IgG1. In certain embodiments, the Fc region comprises amino acid mutations M252Y, S254T, and T256E according to EU numbering. In certain embodiments, the Fc region comprises an amino acid sequence set forth in SEQ ID NO: 36. In certain embodiments, the hinge region comprises an amino acid sequence set forth in SEQ ID NO: 30.

In certain embodiments, the fusion protein comprises, in sequence from the N-terminus to the C-terminus, the human GLP-1 polypeptide variant, the linker, the hinge region, and the immunoglobulin Fc region.

In certain embodiments, the fusion protein comprises an amino acid sequence set forth in any one of SEQ ID NOs: 37-45.

In certain embodiments, the fusion protein comprises an amino acid sequence set forth in any one of SEQ ID NOs: 43-44.

In another aspect, the present application provides an isolated nucleic acid molecule encoding the fusion protein.

In another aspect, the present application provides a vector comprising the isolated nucleic acid molecule.

In another aspect, the present application provides a cell comprising or expressing the fusion protein, the isolated nucleic acid molecule, or the vector.

In another aspect, the present application provides a pharmaceutical composition comprising the fusion protein, the isolated nucleic acid molecule, the vector, and/or the cell, and optionally a pharmaceutically acceptable carrier.

In another aspect, the present application provides an immunoconjugate comprising the fusion protein.

In another aspect, the present application provides use of the fusion protein, the isolated nucleic acid molecule, the vector, the cell, the pharmaceutical composition, and/or the immunoconjugate in preparing a medicament for preventing and/or treating a metabolic disease or condition.

In certain embodiments, the metabolic disease or condition comprises a GLP-1-associated metabolic condition.

In certain embodiments, the metabolic disease or condition comprises diabetes.

In another aspect, the present application provides a method for preventing and/or treating a metabolic disease or condition, which comprises administering to a subject in need thereof the fusion protein, the isolated nucleic acid molecule, the vector, the cell, the pharmaceutical composition, and/or the immunoconjugate.

In certain embodiments, the metabolic disease or condition comprises a GLP-1-associated metabolic condition.

In certain embodiments, the metabolic disease or condition comprises diabetes.

In another aspect, the present application provides the fusion protein, the isolated nucleic acid molecule, the vector, the cell, the pharmaceutical composition, and/or the immunoconjugate for use in preventing and/or treating a metabolic disease or condition.

In certain embodiments, the metabolic disease or condition comprises a GLP-1-associated metabolic condition.

In certain embodiments, the metabolic disease or condition comprises diabetes.

In another aspect, the present application provides a method for increasing or promoting insulin expression in a subject in need thereof, which comprises administering to the subject an effective amount of the fusion protein, the isolated nucleic acid molecule, the vector, the cell, and/or the pharmaceutical composition.

Other aspects and advantages of the present application will be readily apparent to those skilled in the art from the following detailed description. Only exemplary embodiments of the present application are shown and described in the following detailed description. As those skilled in the art will recognize, the content of the present application enables those skilled in the art to make changes to the specific embodiments disclosed without departing from the spirit and scope of the invention involved in the present application. Accordingly, descriptions in the drawings and specification are only illustrative rather than restrictive.

### BRIEF DESCRIPTION OF THE DRAWINGS

Specific features of the invention involved in the present application are set forth in appended claims. Features and advantages of the invention involved in the present application will be better understood by reference to the exemplary embodiments and drawings described in detail below. A brief description of the drawings is as below.
FIG. 1 shows the results of pharmacokinetic detection of the fusion proteins GM-RY-L1H2-Fc4, GM-RY-L2H2-Fc4, and GM-RY-L3H2-Fc4 of the present application with different linkers.
FIG. 2 shows the results of pharmacokinetic detection of the fusion proteins GM-ARY-L2H2-Fc4 and GM-ARY-L2H1-Fc4 of the present application with different hinge regions.
FIG. 3 shows the results of pharmacokinetic detection of the fusion protein GM-RY-L2H1-Fc4m of the present application containing YTE mutation in the Fc region.
FIG. 4 shows the results of pharmacokinetic detection of the fusion proteins GM-ARY-L2H1-Fc4m and GM-RY-L2H1-Fc4m of the present application containing YTE mutation in the Fc region.
FIG. 5 shows the results of pharmacokinetic detection of the fusion proteins GM-ARY-L2H2-Fc4m, GM-RY-L2H2-Fc4m, GM-ARY-L2H1-Fc4m, and GM-RY-L2H1-Fc4m of the present application using different hinge regions and containing YTE mutation.
FIG. 6 shows an example of the amino acid sequence numbering of the human GLP-1 polypeptide variant in a fusion protein of the present application.
FIG. 7 shows the results of the detection that the fusion proteins of the present application are comparable to dulaglutide in the ability to activate GLP-1 receptor.
FIG. 8A shows the binding activity of the fusion proteins with double mutations K26R-W3 1Y, K26R-Q23T, K26R-Q23S, K26R-Q23N, K26R-Y19L, K26R-Y19T, and K26R-Y19F for the GLP-1 receptor.
FIG. 8B shows the binding activity of the fusion proteins with double mutations K26R-W3 1Y, K26R-Q23E, K26R-Y19I, K26R-Y19V, K26R-Y19A, and K26R-Y19S for the GLP-1 receptor.
FIG. 8C shows the binding activity of the fusion proteins with double mutations K26R-W3 1Y, K26R-Q23N, K26R-Q23S, and K26R-Q23T for the GLP-1 receptor.
FIG. 8D shows the binding activity of the fusion proteins with double mutations W31Y-amino acid mutation at position Y19 for the GLP-1 receptor.
FIG. 8E shows the binding activity of the fusion proteins with double amino acid mutations W31Y-Y19A and W31Y-Q23E for the GLP-1 receptor.
FIG. 8F shows the binding activity of fusion proteins GM-WY and GM-WYFL for the GLP-1 receptor.
FIG. 9A shows that both the fusion protein with double mutation K26R-W3 1Y and the fusion protein with triple mutation K26R-W31Y-amino acid mutation at position Q23 can activate the GLP-1 receptor.
FIG. 9B and FIG. 9C show that all the fusion proteins with triple mutations K26R-W31Y-amino acid mutation at position Y19 can all activate the GLP-1 receptor.
FIG. 9D and FIG. 9F show that the fusion proteins with triple mutations K26R-W31Y-amino acid mutation at position Q23 and the fusion proteins with triple mutations K26R-W31Y-amino acid mutation at position Y19 can all activate the GLP-1 receptor. FIG. 9E shows that the fusion proteins with triple mutations K26R-W31Y-amino acid mutation at position Q23 can activate the GLP-1 receptor.
FIG. 10A shows the pharmacokinetic effect of the fusion protein GM-KRWY of the present application.
FIG. 10B shows the pharmacokinetic effect of the fusion proteins GM-KRWYYL, GM-KRWYYA, and GM-KRWYYI of the present application.
FIG. 10C shows the pharmacokinetic effect of the fusion proteins GM-KRWYQN, GM-KRWYQE, and GM-KRWYYA of the present application.
FIGs. 11A-11B show the hypoglycemic activity of the fusion protein GM-KRWY of the present application in an oral glucose tolerance test.
FIGs. 11C-11D show the hypoglycemic activity of the fusion proteins GM-KRWYYA and GM-KRWYQE of the present application in oral glucose tolerance tests.

### DETAILED DESCRIPTION

The embodiments of the invention in the present application are described below with reference to specific examples, and other advantages and effects of the present invention will be readily apparent to those skilled in the art from the disclosure of the present specification.

### Definitions of Terms

In the present application, the term "GLP-1" generally refers to glucagon-like peptide-1. The native GLP-1 molecule undergoes processing *in vivo,* during which the first 6 amino acids are removed by cleaving. Therefore, it is customary in the art to refer to the first amino acid at the N-terminus of the amino acid sequence of GLP-1 as being at position 7 and the last amino acid at the C-terminus as being at position 37. The processed peptide can be further modified *in vivo* by removing the C-terminal glycine residue and replacing it with an amide group. GLP-1 is usually found in two biologically active forms: GLP-1(7-37)OH and GLP-1 (7-36)NH₂. The "GLP-1" described herein includes native, artificially synthesized, or modified GLP-1 proteins and also intact GLP-1 protein or functional fragments thereof, as well as GLP-1 proteins in different biologically active forms. For example, wild-type human GLP-1 may comprise an amino acid sequence set forth in SEQ ID NO: 1, where the N-terminal amino acid residue H is referred to as being at position 7. Accordingly, the term "K26" in the present application generally refers to the position of the amino acid at position 26, counting from the N-terminal H at position 7 of GLP-1 protein; in the amino acid sequence set forth in SEQ ID NO: 1, the amino acid at position 26 is K; the term "W31" generally refers to the position of the amino acid at position 31, counting from the N-terminal H at position 7 of GLP-1 protein; in the amino acid sequence set forth in SEQ ID NO: 1, the amino acid at position 31 is W. In the present application, when describing amino acid mutations and/or substitutions, the numbering of amino acid residues of a GLP-1 polypeptide variant is different from the numbering of amino acid residues in the Fc region, and the numbering of amino acid residues in the Fc region is according to EU numbering. The human GLP-1 may be engineered, and the GLP-1 variant produced by engineering may have at least partial activity of the original human GLP-1. For example, an exemplary engineered wild-type human GLP-1 may have an amino acid sequence set forth in SEQ ID NO: 2.

In the present application, the term "at least partial activity of human GLP-1" generally refers to having one or more activities of human GLP-1 protein or at least 20% (e.g., at least 25%, 30%, 35%, 40%, 45%, or 50% or more) of the activity of the human GLP-1 protein. In the term "at least partial activity of human GLP-1", the human GLP-1 may be the wild type, e.g., the amino acid sequence set forth in SEQ ID NO: 1. In the term "at least partial activity of human GLP-1", the human GLP-1 may be a GLP-1 variant produced by engineering a wild-type human GLP-1, e.g., the amino acid sequence set forth in SEQ ID NO: 2. For example, the human GLP-1 polypeptide variant of the present application may have at least partial activity of human GLP-1 which has an amino acid sequence set forth in SEQ ID NO: 1. In some other cases, the human GLP-1 polypeptide variant of the present application may have at least partial activity of human GLP-1 which has an amino acid sequence set forth in SEQ ID NO: 2.

The activity does not need to be at the same level as the activity of the human GLP-1 protein. It may be similar to, or higher or lower than the activity of the human GLP-1 protein. In certain cases, "at least partial activity of human GLP-1" may refer to one or more selected from the group consisting of: the activity of binding to the GLP-1 receptor, the activity of activating the GLP-1 receptor, the activity of activating adenylate cyclase, the activity of promoting an increase in the intracellular cyclic adenosine monophosphate (cAMP) level, the activity of positively modulating the intracellular Ca²⁺ level, the activity of stimulating insulin secretion, the activity of increasing liver glycogen storage, the activity of delaying gastric emptying, the activity of inhibiting gastric motility, the activity of reducing appetite, the activity of inhibiting β-cell apoptosis, the activity of inhibiting postprandial glucagon secretion, the activity of alleviating hypoglycemia, and the activity of reducing body weight. For example, it can be detected by detecting the ability to bind to the GLP-1 receptor or the level of cAMP expression. For example, it can be detected by luciferase detection of the level of activation of the cAMP/PKA signaling pathway. The "at least partial activity of human GLP-1" may be at least partial activity of a fusion protein that comprises human GLP-1 (e.g., a fusion protein with an Fc region).

In the present application, the term "polypeptide" generally refers to molecules composed of monomers (amino acids) linearly linked by amide bonds (also known as peptide bonds). The term "polypeptide" may refer to any chain of two or more amino acids and does not refer to products of particular length. The polypeptide described herein includes peptides, dipeptides, tripeptides, oligopeptides, proteins, amino acid chains, or any other chains used to refer to having two or more amino acids, and the term "polypeptide" may be used in place of or is used interchangeably with any of these terms. The term "polypeptide" may also refer to the product of post-expression modification of a polypeptide, including but not limited to glycosylation, acetylation, phosphorylation, acylation, derivatization by known protective/blocking groups, proteolytic cleavage, or modification by non-naturally occurring amino acids. The polypeptide may be derived from natural biological sources or produced through recombinant technology.

In the present application, the term "variant" generally refers to a protein molecule having sequence homology with a native biologically active protein. The polypeptide variant described herein includes polypeptides having altered amino acid sequences by addition (including insertion), deletion, modification, and/or substitution of one or more amino acid residues, which differ from the parent sequence (e.g., the amino acid sequence set forth in SEQ ID NO: 2) while retaining at least one therapeutic and/or biological activity of the parent sequence. For example, the variant may have at least 0%, 65%, 70%, 75%, 80%, 85%, 90%, 95%, 96%, 97%, 98%, or 99% sequence identity to the parent protein. The variant may or may not naturally occur. The variant that does not naturally occur can be generated using known techniques in the art. The polypeptide variant of the present invention may comprise conservative or non-conservative amino acid substitutions, deletions, or additions.

In the present application, the term "mutation" generally includes any type of alteration or modification of a sequence (a nucleic acid or an amino acid sequence), including deletion, truncation, inactivation, disruption, substitution, or translocation of an amino acid or a nucleotide. In the present application, an amino acid mutation may be an amino acid substitution, and the term "substitution" generally refers to the replacement of at least one amino acid residue in a predetermined parent amino acid sequence with a different "replacement" amino acid residue. The one or more replaced amino acid residues may be "naturally-occurring amino acid residues" (i.e., genetically encoded).

The term "amino acid substitution" refers to the replacement of one amino acid residue present in a parent sequence with another amino acid residue. Amino acids in the parent sequence may be substituted, for example, via chemical peptide synthesis or by recombinant methods known in the art. In the present application, the term "Fc region" generally refers to a domain derived from the C-terminal region of the heavy chain of an immunoglobulin, and the C-terminal region can be produced by digesting an intact antibody with papain. The Fc region may be an Fc region of a native sequence or an Fc region of a variant, where "immunoglobulin Fc region without the amino acid mutation" refers to the amino acid sequence set forth in any one of SEQ ID NOs: 32-35, which correspond to the Fc sequences of human IgG1, IgG2, IgG3, and IgG4, respectively. The Fc region of the immunoglobulin of the present application typically comprises 2 constant domains (CH₂ domain and CH₃ domain), and as not specifically indicated, does not comprise a hinge region. It may optionally comprise a CH₄ domain.

In the present application, the term "YTE mutation" refers to a group of mutations in the Fc region of IgG. The YTE mutation may result in increased binding of the Fc region to human FcRn and may alter the serum half-life of fusion proteins with an Fc region containing the YTE mutation. The Fc region containing YTE mutation comprises a combination of the following three amino acid mutations: M252Y, S254T, and T256E, where the numbering is according to Kabat EU index.

In the present application, the term "hinge region" generally refers to a flexible amino acid sequence having a length of 15 to 30 amino acids that allows the polypeptide part at its N-terminus or C-terminus to move independently. The hinge region is typically derived from the region between the immunoglobulin heavy chain CH₁ and CH₂ functional regions. The hinge region is generally derived from IgG; for example, it may be derived from IgG1, IgG2, IgG3, or IgG4. The hinge region of the present application may have one or more amino acid residues added to the N-terminus or C-terminus thereof, one or more amino acid residues deleted therefrom, and one or more amino acid residues substituted therein, relative to a native IgG-derived hinge region, so long as the function of the hinge region is maintained.

In the present application, the term "immunoglobulin" generally refers to a protein consisting essentially of one or more polypeptides encoded by immunoglobulin genes. Recognized human immunoglobulin genes include κ, λ, α (IgA1 and IgA2), γ (IgG1, IgG2, IgG3, and IgG4), δ, ε, and µ constant region genes, as well as a multitude of immunoglobulin variable region genes. Generally, the immunoglobulin may be a heterotetrameric glycoprotein of about 150,000 daltons consisting of two identical light (L) chains and two identical heavy (H) chains. Of the "light chain" (about 25 KD and 214 amino acids) of a full-length immunoglobulin, the NH2-terminus (about 110 amino acids) is encoded by a variable region gene and the COOH-terminus is encoded by a κ or λ constant region gene. A native immunoglobulin consists essentially of two Fab molecules and an Fc region linked by an immunoglobulin hinge region.

In the present application, the term "linker" generally refers to an amino acid sequence that links two heterologous polypeptides or fragments thereof. In the present application, the linker may be an amino acid sequence that covalently links the polypeptides to form a fusion polypeptide. The linker may have a length of 10-20 amino acids. The linker may be flexible or rigid.

In the present application, the "selectively enhance" generally means enhancement under specific conditions, which may be a specific pH range, a specific temperature range, a specific linking mode, a specific sample source, a specific reaction time, or the like. For example, amino acid mutations in the Fc region result in increased binding of the Fc region to an Fc receptor within a particular pH range, but do not significantly alter the binding of the Fc region to the Fc receptor under a pH that is outside that particular range, and this may be referred to as "selectively enhance".

In the present application, the term "fusion protein" generally refers to a longer polypeptide comprising or consisting of a polypeptide whose amino acid sequence can be fused directly or indirectly (via a linker, e.g., a linker peptide) to the amino acid sequence of a heterologous polypeptide (e.g., a polypeptide unrelated to the previous polypeptide or the domain thereof).

In the present application, the term "immunoconjugate" generally refers to a conjugate formed by directly or indirectly linking (e.g., covalently via a linker) a polypeptide or protein to an active substance of interest (e.g., a protein, a nucleic acid, a drug, or a label molecule).

In the present application, the term "nucleic acid molecule" generally refers to nucleotides, deoxyribonucleotides or ribonucleotides or analogs thereof in an isolated form and of any length that are isolated from its natural environment or artificially synthesized.

In the present application, the term "vector" generally refers to a nucleic acid molecule capable of self-replication in a suitable host cell, which transfers an inserted nucleic acid molecule into a host cell and/or between host cells. The vector may include a vector for primarily inserting DNA or RNA into a cell, a vector for primarily replicating DNA or RNA, and a vector for primarily expressing transcription and/or translation of DNA or RNA. The vector may also include vectors having a variety of the above-described functions. The vector may be a polynucleotide capable of being transcribed and translated into a polypeptide when introduced into a suitable host cell. Generally, the vector can produce the desired expression product by culturing an appropriate host cell comprising the vector. In the present application, the term "cell" generally refers to an individual cell, cell line, or cell culture that may comprise or has comprised a plasmid or vector comprising the nucleic acid molecule described herein, or that is capable of expressing the antibody or the antigen-binding fragment thereof described herein. The host cell may comprise progeny of a single host cell. Due to natural, accidental, or deliberate mutations, progeny cells may not necessarily be identical in morphology or in genome to the original parent cell, but are capable of expressing the antibody or the antigen-binding fragment thereof described herein. The host cell may be obtained by transfecting cells with the vector described herein *in vitro.* The host cell may be a prokaryotic cell (e.g., *E. coli*) or a eukaryotic cell (e.g., a yeast cell, a COS cell, a Chinese hamster ovary (CHO) cell, a HeLa cell, an HEK293 cell, a COS-1 cell, an NS0 cell, or a myeloma cell). The recombinant host cell includes not only particular cells but also progeny of such cells.

In the present application, the term "metabolic disease or condition" generally refers to a disease or condition that disrupts the normal metabolic processes in the body and thereby renders the body unable to properly utilize and/or store energy. The metabolic disease or condition may refer to diseases of carbohydrate metabolism disorders, diseases of lipid metabolism disorders, and/or diseases of mitochondrial disorders. The metabolic disease or condition may be associated with diet, toxins, or infections, or may be a genetic disease. The metabolic disease or condition may include diseases or conditions caused by lack of metabolism-associated enzymes or metabolism-associated enzymatic dysfunction. The metabolic disease or condition described herein may be selected from diabetes and other GLP-1-associated metabolic conditions.

In the present application, "dulaglutide" generally refers to a heterologous fusion protein comprising a GLP-1 analog, which is formed by covalently linking the GLP-1 analog (the amino acid sequence is set forth in SEQ ID NO: 2) to an Fc region derived from human IgG4 via a small-molecule peptide. Dulaglutide is sold under the brand name TRULICITY^{®}. Dulaglutide comprises an amino acid sequence set forth in SEQ ID NO: 3 (see PCT patent WO2009009562 and U.S. Pat. US7452966).

In the present application, the term "comprise", "comprising", "contain", or "containing" is generally intended to include the explicitly specified features without excluding other elements.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### Detailed Description of the Invention

### GLP-1 polypeptide variant

In one aspect, the present application provides a fusion protein comprising a human GLP-1 polypeptide variant having at least partial activity of human GLP-1. In certain cases, the human GLP-1 polypeptide variant may have one or more activities selected from the group consisting of: the activity of binding to the GLP-1 receptor, the activity of activating the GLP-1 receptor, the activity of activating adenylate cyclase, the activity of promoting an increase in the intracellular cyclic adenosine monophosphate (cAMP) level, the activity of positively modulating the intracellular Ca²⁺ level, the activity of stimulating insulin secretion, the activity of increasing liver glycogen storage, the activity of delaying gastric emptying, the activity of inhibiting gastric motility, the activity of reducing appetite, the activity of inhibiting β-cell apoptosis, the activity of inhibiting postprandial glucagon secretion, the activity of alleviating hypoglycemia, and the activity of reducing body weight. For example, it can be detected by detecting the ability to bind to the GLP-1 receptor or the level of cAMP expression. The activity of the human GLP-1 polypeptide variant may be at least 20% (e.g., at least 25%, 30%, 35%, 40%, 45%, or 50% or more) of the activity of human GLP-1.

The amino acid sequence of the human GLP-1 polypeptide variant may comprise at least 2 amino acid mutations, e.g., 2, 3, or more amino acid mutations, compared with the amino acid sequence set forth in SEQ ID NO: 2: HGEGTFTSDVSSYLEEQAAKEFIAWLVKGGG (SEQ ID NO: 2). For example, the amino acid sequence of the human GLP-1 polypeptide variant may comprise 2-4 (e.g., 2-3, 3, or 2) amino acid substitutions compared with the amino acid sequence set forth in SEQ ID NO: 2. The at least 2 amino acid mutations may be at amino acid positions selected from the group consisting of K26, W31, and Y19.

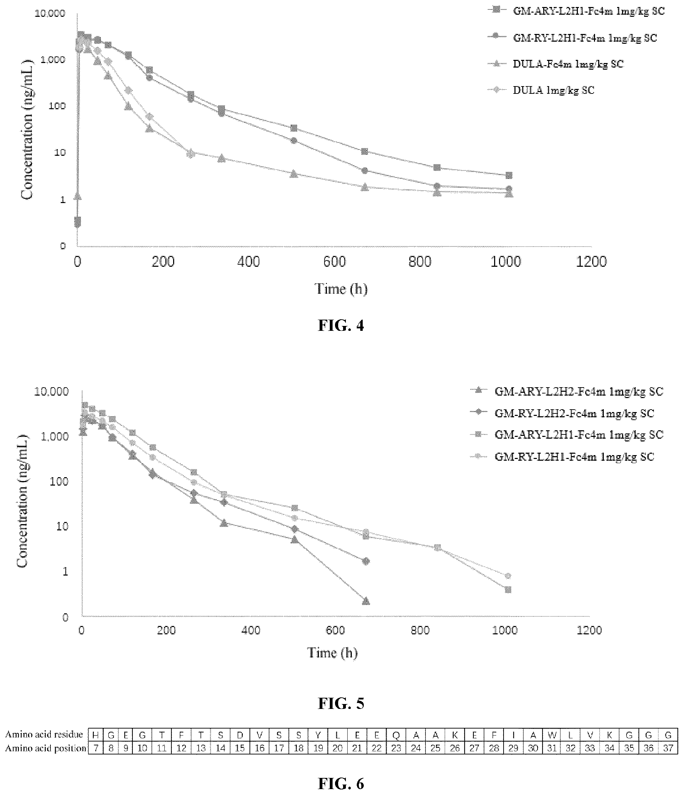

In the present application, the amino acid position "Xn" means that an amino acid substitution occurs at residue X corresponding to position n in the amino acid sequence set forth in SEQ ID NO: 1 or SEQ ID NO: 2, where n is a positive integer (for GLP-1, n starts at 7) and X is the abbreviation of any amino acid residue. For example, amino acid position "W31" indicates the position corresponding to the amino acid at position 31 of the amino acid sequence set forth in SEQ ID NO: 1 or SEQ ID NO: 2. For the amino acid sequence set forth in SEQ ID NO: 2, the correspondence of amino acid residue X to position n is shown in FIG. 6.

In the present application, the amino acid sequence of the human GLP-1 polypeptide variant may comprise at least 2 mutations at amino acid positions selected from the group consisting of W31, K26, and Y19 compared with the amino acid sequence set forth in SEQ ID NO: 2.

For example, the amino acid sequence of the human GLP-1 polypeptide variant may comprise 2 amino acid mutations at amino acid positions W31 and K26 compared with the amino acid sequence set forth in SEQ ID NO: 2. For example, the amino acid sequence of the human GLP-1 polypeptide variant has 2 amino acid mutations, which may be at amino acid positions W31 and K26. For example, the human GLP-1 polypeptide variant may comprise an amino acid sequence set forth in SEQ ID NO: 4.

For example, the amino acid sequence of the human GLP-1 polypeptide variant may comprise 2 amino acid mutations at amino acid positions W31 and Y19 compared with the amino acid sequence set forth in SEQ ID NO: 2. For example, the amino acid sequence of the human GLP-1 polypeptide variant has 2 amino acid mutations, which may be at amino acid positions W31 and Y19. For example, the human GLP-1 polypeptide variant may comprise an amino acid sequence set forth in any one of SEQ ID NOs: 12-18.

For example, the amino acid sequence of the human GLP-1 polypeptide variant may comprise 2 amino acid mutations at amino acid positions K26 and Y19 compared with the amino acid sequence set forth in SEQ ID NO: 2. For example, the amino acid sequence of the human GLP-1 polypeptide variant has 2 amino acid mutations, which may be at amino acid positions K26 and Y19. For example, the human GLP-1 polypeptide variant may comprise an amino acid sequence set forth in any one of SEQ ID NOs: 5-11.

For example, the amino acid sequence of the human GLP-1 polypeptide variant may comprise 3 amino acid mutations at amino acid positions W31, K26, and Y19 compared with the amino acid sequence set forth in SEQ ID NO: 2. For example, the amino acid sequence of the human GLP-1 polypeptide variant has 3 amino acid mutations, which may be at amino acid positions W31, K26, and Y19. For example, the human GLP-1 polypeptide variant may comprise an amino acid sequence set forth in any one of SEQ ID NOs: 19-25.

In the present application, the amino acid mutations may include amino acid substitutions. The amino acid mutations cause the GLP-1 polypeptide variant to retain at least partial activity of human GLP-1 (e.g., a human GLP-1 protein comprising an amino acid sequence set forth in any one of SEQ ID NOs: 1 and 2). The amino acid mutations may cause a fusion protein of the GLP-1 polypeptide variant to retain at least partial activity of a fusion protein of human GLP-1 (e.g., a human GLP-1 protein comprising an amino acid sequence set forth in any one of SEQ ID NOs: 1 and 2). The amino acid mutations may cause a fusion protein of the GLP-1 polypeptide variant and an Fc region to retain at least partial activity of a fusion protein of human GLP-1 (e.g., a human GLP-1 protein comprising an amino acid sequence set forth in any one of SEQ ID NOs: 1 and 2) and the Fc region.

In the present application, an amino acid substitution may be comprised at the position K26, and the amino acid substitution may be K26R.

In the present application, an amino acid substitution may be comprised at the position W31, and the amino acid substitution may be W31Y, W31A, W31R, or W31K. In the present application, an amino acid substitution may be comprised at the position W31, and the amino acid substitution may be W31Y, W31K, or W31R. For example, an amino acid substitution may be comprised at the position W31, and the amino acid substitution may be W31Y.

In the present application, an amino acid substitution may be comprised at the position Y19, and the amino acid substitution may be Y19A, Y19L, Y19T, Y19F, Y19I, Y19V, or Y19S. For example, an amino acid substitution may be comprised at the position Y19, and the amino acid substitution may be Y19A.

In the present application, the amino acid substitution "XnY" means that residue X corresponding to position n in the amino acid sequence set forth in SEQ ID NO: 1 or SEQ ID NO: 2 is substituted with amino acid residue Y, where n is a positive integer (for GLP-1, n starts at 7); X and Y are each independently the abbreviation of any amino acid residue and X differs from Y. For example, the amino acid substitution "W31Y" means that the amino acid residue W corresponding to position 31 in the amino acid sequence set forth in SEQ ID NO: 1 or SEQ ID NO: 2 is substituted with the amino acid residue Y.

In the present application, the amino acid sequence of the human GLP-1 polypeptide variant may comprise at least 2 amino acid substitutions compared with the amino acid sequence set forth in SEQ ID NO: 1 or SEQ ID NO: 2, and the amino acid substitutions may be selected from the group consisting of: 1) K26R, 2) any one selected from W31Y, W31R, W31K, and W31A, and 3) any one selected from Y19A, Y19L, Y19T, Y19F, Y19I, Y19V, and Y19S.

For example, compared with the amino acid sequence set forth in SEQ ID NO: 2, the amino acid sequence of the human GLP-1 polypeptide variant may comprise at least 2 amino acid mutations selected from the group consisting of: 1) K26R, 2) any one selected from W31Y, W31R, W31K, and W31A, and 3) any one selected from Y19A, Y19L, Y19T, Y19F, Y19I, Y19V, and Y19S.

In the present application, the amino acid sequence of the human GLP-1 polypeptide variant may comprise 2 amino acid substitutions at amino acid positions W31 and K26 compared with the amino acid sequence set forth in SEQ ID NO: 2, where the amino acid substitution at the position K26 may be K26R, and the amino acid substitution at the position W31 may be W31Y, W31R, W31K, and W31A. For example, the amino acid sequence of the human GLP-1 polypeptide variant may comprise amino acid substitutions W31Y and K26R compared with the amino acid sequence set forth in SEQ ID NO: 2.

In the present application, the amino acid sequence of the human GLP-1 polypeptide variant may comprise amino acid substitution W31Y compared with the amino acid sequence set forth in SEQ ID NO: 2.

In the present application, the amino acid sequence of the human GLP-1 polypeptide variant may comprise amino acid substitution K26R compared with the amino acid sequence set forth in SEQ ID NO: 2.

In the present application, the amino acid sequence of the human GLP-1 polypeptide variant may comprise amino acid substitution Y19A compared with the amino acid sequence set forth in SEQ ID NO: 2.

In the present application, the amino acid sequence of the human GLP-1 polypeptide variant may comprise amino acid substitutions W31Y and Y19A compared with the amino acid sequence set forth in SEQ ID NO: 2.

In the present application, the amino acid sequence of the human GLP-1 polypeptide variant may comprise amino acid substitutions W31Y and K26R compared with the amino acid sequence set forth in SEQ ID NO: 2. For example, the human GLP-1 polypeptide variant may comprise an amino acid sequence set forth in any one of SEQ ID NOs: 4 and 19-25.

For example, the amino acid substitutions in the amino acid sequence of the human GLP-1 polypeptide variant may be W31Y and K26R. For example, the human GLP-1 polypeptide variant may comprise an amino acid sequence set forth in SEQ ID NO: 4.

In the present application, the amino acid sequence of the human GLP-1 polypeptide variant may comprise amino acid substitution K26R, amino acid substitution W31Y, and an amino acid substitution at position Y19 compared with the amino acid sequence set forth in SEQ ID NO: 2. For example, the GLP-1 polypeptide variant may comprise an amino acid sequence set forth in any one of SEQ ID NOs: 19-25.

In the present application, the amino acid sequence of the human GLP-1 polypeptide variant may comprise amino acid substitution K26R, amino acid substitution W31Y, and amino acid substitution Y19A compared with the amino acid sequence set forth in SEQ ID NO: 2. For example, the human GLP-1 polypeptide variant may comprise an amino acid sequence set forth in SEQ ID NO: 25.

In the present application, the human GLP-1 polypeptide variant may comprise an amino acid sequence set forth in SEQ ID NO: 26: HGEGTFTSDVSSX₁₉LEEQAAREFIAX₃₁LVKGGG, where X₁₉ = Y, A, L, T, F, I, V, or S, and X₃₁ = W, A, R, K, or Y.

PCT International patent applications PCT/CN2021/141606 and PCT/CN2021/141608 relate to human GLP-1 polypeptide variants and fusion proteins thereof and are incorporated herein by reference in their entireties.

### Fc region

In the present application, the fusion protein comprises an immunoglobulin Fc region. In the present application, the immunoglobulin Fc region may be derived from IgG, e.g., from IgG1, IgG2, IgG3, or IgG4. The Fc region of IgG1 may comprise an amino acid sequence set forth in SEQ ID NO: 32. The Fc region of IgG2 may comprise an amino acid sequence set forth in SEQ ID NO: 33. The Fc region of IgG3 may comprise an amino acid sequence set forth in SEQ ID NO: 34. The Fc region of IgG4 may comprise an amino acid sequence set forth in SEQ ID NO: 35.

In the present application, the Fc region may comprise an amino acid mutation, and the amino acid mutation may selectively enhance binding affinity of the immunoglobulin Fc region for an Fc receptor compared with an immunoglobulin Fc region without the amino acid mutation (e.g., an amino acid sequence set forth in any one of SEQ ID NOs: 32-35).

In the present application, the Fc region may be an Fc region derived from IgG1, IgG2, IgG3, or IgG4, and comprises one or more amino acid mutations at positions M252, S254, and/or T256 (e.g., the amino acid positions are according to Kabat EU numbering). For example, the Fc region may be an Fc region derived from IgG1, IgG2, IgG3, or IgG4, and comprises amino acid mutations at positions M252, S254, and T256 (e.g., the amino acid positions are according to Kabat EU numbering).

For example, the amino acid mutation at M252 may be M252Y. For example, the amino acid mutation at S254T may be S254T. For example, the amino acid mutation at T256 may be T256E.

In the present application, the Fc region may be an Fc region derived from IgG1, IgG2, IgG3, or IgG4, and may comprise amino acid mutations M252Y, S254T, and T256E.

For example, the Fc region may be an Fc region derived from IgG1, and may comprise amino acid mutations M252Y, S254T, and T256E. For example, the Fc region may be an Fc region derived from IgG2, and may comprise amino acid mutations M252Y, S254T, and T256E. For example, the Fc region may be an Fc region derived from IgG3, and may comprise amino acid mutations M252Y, S254T, and T256E. For example, the Fc region may be an Fc region derived from IgG4, and may comprise an amino acid sequence set forth in SEQ ID NO: 36.

### Hinge region

In the present application, the fusion protein may comprise a hinge region. In the present application, the hinge region may be derived from IgG.

In the present application, the hinge region may be derived from IgG1, IgG2, IgG3, or IgG4. In the present application, the hinge region may be derived from IgG1. In the present application, the hinge region may be derived from IgG4.

In the present application, compared with a naturally occurring IgG1 hinge region, the hinge region in the fusion protein may comprise one or more amino acid mutations to remove inter-chain disulfide bonds. Such amino acid mutations are known techniques in the art.

In the present application, compared with a naturally occurring IgG hinge region, the hinge region in the fusion protein may have one or more amino acids added at the N-terminus. In the present application, compared with a naturally occurring IgG hinge region, the hinge region in the fusion protein may have one or more amino acids added at the C-terminus. In the present application, compared with a naturally occurring IgG hinge region, the hinge region in the fusion protein may have one or more amino acids deleted at the N-terminus. In the present application, compared with a naturally occurring IgG hinge region, the hinge region in the fusion protein may have one or more amino acids deleted at the C-terminus.

In the present application, compared with a naturally occurring IgG4 hinge region, the hinge region in the fusion protein may have one or more amino acids, e.g., Ala, added at the N-terminus. In the present application, compared with a naturally occurring IgG4 hinge region, the hinge region in the fusion protein may comprise one or more amino acid mutations to avoid undesirable protein formation. Such amino acid mutations are known techniques in the art.

In the present application, the hinge region may comprise an amino acid sequence set forth in SEQ ID NO: 30 or 31.

### Linker

In the present application, the fusion protein may comprise a linker. The linker links the human GLP-1 polypeptide variant and the immunoglobulin Fc region. In the present application, the linker may be a peptide linker. In the present application, the peptide linker may be rigid or flexible. Exemplary linkers may include, but are not limited to, an amino acid sequence set forth in any one of SEQ ID NOs: 27-29 or 47-70.

In the present application, the linker may have a length of 12-15 amino acids. In the present application, the linker may have a length of 15 amino acids, and for the linkers less than 15 amino acids in length as exemplified above, the length thereof may be increased to 15 amino acids by adding amino acids, e.g., Gly.

For example, the linker described herein may comprise an amino acid sequence set forth in any one of SEQ ID NOs: 27-29.

### Fusion protein

In the present application, the fusion protein may comprise the human GLP polypeptide variant, the linker, the hinge region, and the immunoglobulin Fc region.

In the present application, the human GLP polypeptide variant in the fusion protein may comprise an amino acid sequence set forth in SEQ ID NO: 4 or 25 and the immunoglobulin Fc region may be an Fc region derived from IgG. For example, the immunoglobulin Fc region may be an Fc region of IgG comprising amino acid mutations M252Y, S254T, and T256E.

In the present application, the human GLP polypeptide variant in the fusion protein may comprise an amino acid sequence set forth in SEQ ID NO: 4 or 25, the hinge region may be derived from the hinge region of IgG1 or IgG4, and the immunoglobulin Fc region may be an Fc region derived from IgG. For example, the immunoglobulin Fc region may be an Fc region of IgG comprising amino acid mutations M252Y, S254T, and T256E.

In the present application, the human GLP polypeptide variant in the fusion protein may comprise an amino acid sequence set forth in SEQ ID NO: 4 or 25, the linker may comprise an amino acid sequence set forth in any one of SEQ ID NOs: 27-29, the hinge region may be derived from the hinge region of IgG1 or IgG4, and the immunoglobulin Fc region may be an Fc region derived from IgG. For example, the immunoglobulin Fc region may be an Fc region of IgG comprising amino acid mutations M252Y, S254T, and T256E.

In the present application, the fusion protein may comprise, in sequence from the N-terminus to the C-terminus, the human GLP polypeptide variant, the linker, the hinge region, and the immunoglobulin Fc region.

In the present application, the human GLP polypeptide variant in the fusion protein may comprise an amino acid sequence set forth in SEQ ID NO: 4, the linker may comprise an amino acid sequence set forth in any one of SEQ ID NOs: 27-29, the hinge region may be derived from the hinge region of IgG1 or IgG4, and the immunoglobulin Fc region may be an Fc region derived from IgG4. For example, the fusion protein may comprise an amino acid sequence set forth in any one of SEQ ID NOs: 37-39.

In the present application, the human GLP polypeptide variant in the fusion protein may comprise an amino acid sequence set forth in SEQ ID NO: 25, the linker may comprise an amino acid sequence set forth in SEQ ID NO: 28, the hinge region may be derived from the hinge region of IgG1 or IgG4, and the immunoglobulin Fc region may be an Fc region derived from IgG4 and optionally comprises amino acid mutations M252Y, S254T, and T256E. For example, the fusion protein may comprise an amino acid sequence set forth in any one of SEQ ID NOs: 40-42 and 44.

In the present application, the human GLP polypeptide variant in the fusion protein may comprise an amino acid sequence set forth in SEQ ID NO: 4, the linker may comprise an amino acid sequence set forth in SEQ ID NO: 28, the hinge region may be derived from the hinge region of IgG1 or IgG4, and the immunoglobulin Fc region may be an Fc region derived from IgG4 and comprises amino acid mutations M252Y, S254T, and T256E. For example, the fusion protein may comprise an amino acid sequence set forth in any one of SEQ ID NOs: 43 and 45.

In the present application, the human GLP polypeptide variant in the fusion protein may comprise an amino acid sequence set forth in SEQ ID NO: 4, the linker may comprise an amino acid sequence set forth in SEQ ID NO: 28, the hinge region may be derived from the hinge region of IgG1, and the immunoglobulin Fc region may be an Fc region derived from IgG4 and comprises amino acid mutations M252Y, S254T, and T256E. For example, the fusion protein may comprise an amino acid sequence set forth in SEQ ID NO: 43.

In the present application, the human GLP polypeptide variant in the fusion protein may comprise an amino acid sequence set forth in SEQ ID NO: 25, the linker may comprise an amino acid sequence set forth in SEQ ID NO: 28, the hinge region may be derived from the hinge region of IgG1, and the immunoglobulin Fc region may be an Fc region derived from IgG4 and comprises amino acid mutations M252Y, S254T, and T256E. For example, the fusion protein may comprise an amino acid sequence set forth in SEQ ID NO: 44.

### Nucleic acid molecule, vector, cell and preparation method

In another aspect, the present application also provides one or more isolated nucleic acid molecules capable of encoding the fusion protein described herein. For example, each of the one or more nucleic acid molecules may encode an intact fusion protein or may encode a portion thereof.

The nucleic acid molecules described herein may be isolated. For example, it may be produced or synthesized by the following methods: (i) *in vitro* amplification, such as amplification by polymerase chain reaction (PCR); (ii) cloning and recombination; (iii) purification, such as separation by enzymatic digestion and gel electrophoresis fractionation; or (iv) synthesis, such as chemical synthesis. In certain embodiments, the isolated nucleic acid is a nucleic acid molecule prepared by recombinant DNA techniques. Recombinant DNA and molecular cloning techniques include those described in Sambrook, J., Fritsch, E. F. and Maniatis, T. Molecular Cloning: A Laboratory Manual; Cold Spring Harbor Laboratory Press: Cold Spring Harbor, (1989) (Maniatis) and T. J. Silhavy, M. L. Bennan and L. W. Enquist, Experiments with Gene Fusions, Cold Spring Harbor Laboratory, Cold Spring Harbor, N.Y. (1984), as well as Ausubel, F. M. et al., Current Protocols in Molecular Biology, pub. by Greene Publishing Assoc. and Wiley-Interscience (1987). Briefly, the nucleic acids can be prepared from genomic DNA fragments, cDNA and RNA, and all of the nucleic acids can be directly extracted from cells or recombinantly produced by various amplification methods, including but not limited to PCR and RT-PCR.

In another aspect, the present application provides one or more vectors comprising the nucleic acid molecules. For example, the vector may comprise one or more of the nucleic acid molecules. In addition, the vector may further comprise other genes, such as marker genes that allow selection of the vector in an appropriate host cell and under appropriate conditions. In addition, the vector may further comprise expression control elements that allow proper expression of the coding region in an appropriate host. Such control elements are well known to those skilled in the art and may include, for example, promoters, ribosome binding sites, enhancers, and other control elements for regulating gene transcription or mRNA translation. The one or more nucleic acid molecules described herein may be operably linked to the expression control elements.

The vector may include, for example, plasmids, cosmids, viruses, phages, or other vectors commonly used in, for example, genetic engineering. For example, the vector is an expression vector.

In another aspect, the present application provides a cell that may comprise the one or more nucleic acid molecules described herein and/or the one or more vectors described herein. In certain embodiments, each type of or each host cell may comprise one or one type of nucleic acid molecule or vector described herein. In certain embodiments, each type of or each host cell may comprise multiple (e.g., two or more) or multiple types (e.g., two or more) of nucleic acid molecules or vectors described herein. For example, the vector described herein can be introduced into the cell, e.g., a prokaryotic cell (e.g., a bacterial cell), a CHO cell, an NS0 cell, an HEK293T cell, or an HEK293A cell, or other eukaryotic cells, such as a plant-derived cell, a fungal or yeast cell, or the like. The vector described herein can be introduced into the cell by methods known in the art, such as electroporation and liposomal transfection.

In another aspect, the present application provides a method for preparing the fusion protein described herein. The method may comprise culturing the cell described herein under conditions that allow expression of the fusion protein, for example, by adopting an appropriate medium, an appropriate temperature, an appropriate culture time, and the like.

In certain cases, the method may further comprise the step of harvesting (e.g., isolating and/or purifying) the fusion protein described herein. For example, affinity chromatography can be performed with protein G-agarose or protein A-agarose; the fusion protein described herein can also be purified and isolated by gel electrophoresis and/or high performance liquid chromatography, etc.

### Pharmaceutical composition and use

In another aspect, the present application provides a pharmaceutical composition comprising the fusion protein, the nucleic acid molecule, the vector, or the cell, and optionally a pharmaceutically acceptable adjuvant.

For example, the pharmaceutically acceptable adjuvant may include buffer, antioxidant, preservative, low molecular weight polypeptide, protein, hydrophilic polymer, amino acid, sugar, chelating agent, counter ion, metal complex, and/or nonionic surfactant, etc.

In the present application, the pharmaceutical composition may be formulated for oral administration, intravenous administration, intramuscular administration, *in situ* administration at the tumor site, inhalation, rectal administration, vaginal administration, transdermal administration, or administration via a subcutaneous depot.

The pharmaceutical composition described herein can be used to treat a metabolic disease or condition. For example, the metabolic disease or condition may be selected from diabetes and other GLP-1-associated metabolic conditions.

The frequency of administration and the dose of the pharmaceutical composition can be determined by a variety of relevant factors, including the type of the disease to be treated, the route of administration, the age, sex and body weight of the patient and the severity of the disease, as well as the type of the drug used as an active ingredient. Since the pharmaceutical composition has excellent durations of *in vivo* efficacy and concentration, it can significantly reduce the frequency of administration and the dose of the drug.

In another aspect, the present application provides a method for increasing or promoting insulin expression in a subject in need thereof, which comprises administering to the subject an effective amount of the fusion protein, the isolated nucleic acid molecule, the vector, the cell, and/or the pharmaceutical composition.

Without being bound by any theory, the following examples are intended only to illustrate various technical solutions of the invention in the present application, but not to limit the scope of the present application.

### Examples

### Example 1. Preparation of Fusion Proteins

Fusion proteins of a human GLP-1 polypeptide variant and an Fc were prepared, and the fusion proteins comprised, in sequence from the N-terminus to the C-terminus, a GLP-1 polypeptide variant (the amino acid sequence is set forth in any one of SEQ ID NOs: 4-25), a linker peptide (the amino acid sequence is set forth in any one of SEQ ID NOs: 27-29), a hinge region (the amino acid sequence is set forth in any one of SEQ ID NOs: 30-31), and an Fc region derived from IgG (the amino acid sequence is set forth in any one of SEQ ID NOs: 32-36). Vectors comprising nucleic acid sequences encoding the fusion proteins were transfected into cells, and the fusion proteins were obtained by expression and purification. The full-length sequences and partial sequences of exemplary fusion proteins are shown in Table 1 below.

**Table 1. Structures and amino acid sequences of exemplary fusion proteins**

| Fusion protein | Linking mode | Full-length sequence (SEQ ID NO) | Human GLP polypeptide variant (SEQ ID NO) | Linker (SEQ ID NO) | Hinge region (SEQ ID NO) | Fc region (SEQ ID NO) |
|---|---|---|---|---|---|---|
| GM-RY-L1H2-Fc4 | GLP-1 with double mutations (K26R W31Y) + linker L1 + hinge region H2 + Fc region of IgG4 | 37 | 4 | 27 | 31 | 35 |
| GM-RY-L2H2-Fc4 | GLP-1 with double mutations (K26R W31Y) + linker L2 + hinge region H2 + Fc region of IgG4 | 38 | 4 | 28 | 31 | 35 |
| GM-RY-L3H2-Fc4 | GLP-1 with double mutations (K26R W31Y) + linker L3 + hinge region H2 + Fc region of IgG4 | 39 | 4 | 29 | 31 | 35 |
| GM-ARYL2H2-Fc4 | GLP-1 with triple mutations (Y19A K26R W31Y) + linker L2 + hinge region H2 + Fc region of IgG4 | 40 | 25 | 28 | 31 | 35 |
| GM-ARYL2H1-Fc4 | GLP-1 with triple mutations (Y19A K26R W31Y) + linker L2 + hinge region H1 + Fc region of IgG4 | 41 | 25 | 28 | 30 | 35 |
| DULA | Dulaglutide | 3 | - | - | - | - |
| DULA-Fc4m | Dulaglutide (Fc region of IgG4 with YTE mutation) | 46 | - | - | - | - |
| GM-RY-L2H1-Fc4m | GLP-1 with double mutations (K26R W31Y) + linker L2 + hinge region H1 + Fc region of IgG4 with YTE mutation | 43 | 4 | 28 | 30 | 36 |
| GM-ARYL2H1-Fc4m | GLP-1 with triple mutations (Y19A K26R W31Y) + linker L2 + hinge region H1 + Fc region of IgG4 with YTE mutation | 44 | 25 | 28 | 30 | 36 |
| GM-RY-L2H2-Fc4m | GLP-1 with double mutations (K26R W31Y) + linker L2 + hinge region H2 + Fc region of IgG4 with YTE mutation | 45 | 4 | 28 | 31 | 36 |
| GM-ARYL2H2-Fc4m | GLP-1 with triple mutations (Y19A K26R W31Y) + linker L2 + hinge region H2 + Fc region of IgG4 with YTE mutation | 42 | 25 | 28 | 31 | 36 |

### Example 2. Pharmacokinetic Detection of Fusion Proteins Using Different Linkers

The *in vivo* pharmacokinetics of the fusion proteins GM-RY-L1H2-Fc4, GM-RY-L2H2-Fc4, and GM-RY-L3H2-Fc4 of the present application were examined. The experiment was carried out in an SPF animal room at an ambient temperature of 23±2 °C and 40-70% relative humidity, with alternate light and darkness (12 h). The experimental animals were freely fed, and before the experiment, acclimated for at least 3 days. SPF SD rats weighing 180-220 g were randomly grouped. Rats in each group were subcutaneously injected (SC) with a corresponding test sample at a dose of 1 mg/kg, and dulaglutide (DULA) was used as a control. Blood samples of about 100 µL were taken from the jugular vein before administration and 6 h, 24 h, 48 h, 72 h, 96 h, and 120 h after administration. The blood samples were added to centrifuge tubes and left to stand at room temperature for 30-60 min. The blood was then centrifuged at 4 °C at 4000 rpm for 10 min within 2 h, and the serum was rapidly isolated and stored at -80 °C. The samples were taken for ELISA analysis. The pharmacokinetic parameters were calculated using DAS (3.2.8) software.

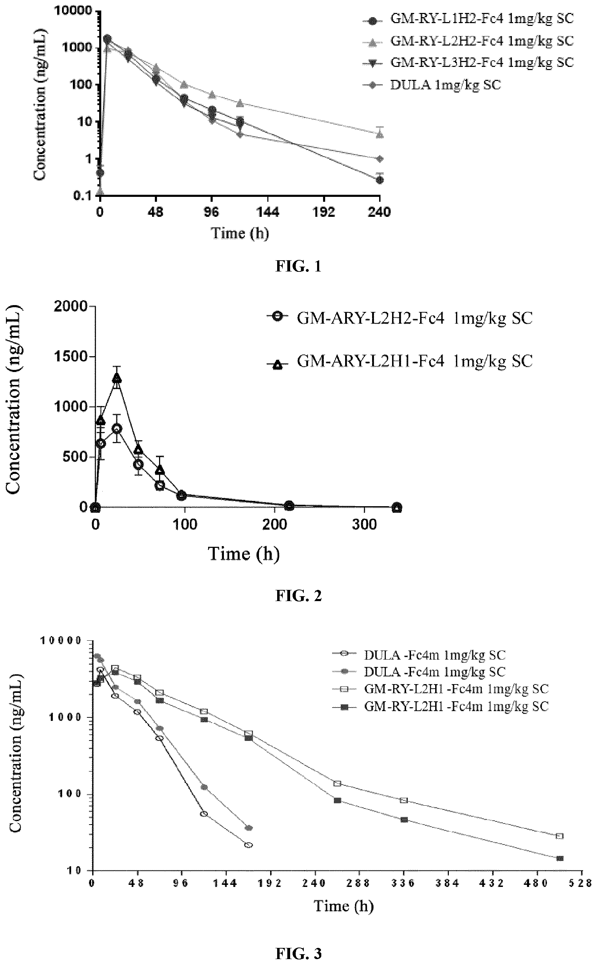

The results are shown in FIG. 1 and Table 2. The *in vivo* metabolic properties of GM-RY-L1H2-Fc4, GM-RY-L2H2-Fc4, and GM-RY-L3H2-Fc4 were not significantly different from those of the control dulaglutide, where GM-RY-L2H2-Fc4 using the L2 linker (SEQ ID NO: 28) had the best half-life and its exposure was comparable to that of the control.

**Table 2. Results of pharmacokinetic detection**

| Parameter | AUC(0-t) ug/L*h | t1/2 h | Tmax h | Cmax ug/L |
|---|---|---|---|---|
| DULA 1mg/kg SC | 43954.547 | 13.671 | 6 | 1579.77 |
| GM-RY-L1H2-Fc4 1mg/kg SC | 42673.641 | 16.199 | 6 | 1851.87 |
| GM-RY-L2H2-Fc4 1mg/kg SC | 41231.843 | 32.501 | 6 | 984.447 |
| GM-RY-L3H2-Fc4 1mg/kg SC | 31916.805 | 16.077 | 6 | 1402.199 |

### Example 3. Pharmacokinetic Detection of Fusion Proteins Using Different Hinge Regions

The *in vivo* pharmacokinetics of the fusion proteins GM-ARY-L2H2-Fc4 and GM-ARY-L2H1-Fc4 of the present application were detected according to the method in Example 2. The samples were taken for ELISA analysis. The pharmacokinetic parameters were calculated using DAS (3.2.8) software.

The results are shown in FIG. 2 and Table 3. GM-ARY-L2H2-Fc4 and GM-ARY-L2H1-Fc4 were comparable in *in vivo* half-life, and GM-ARY-L2H1-Fc4 using the hinge region of IgG1 (SEQ ID NO: 30) had better exposure.

**Table 3. Results of pharmacokinetic detection**

| Parameter | AUC(0-t) | t1/2 | Tmax | Cmax |
|---|---|---|---|---|
| | ug/L*h | h | h | ug/L |
| GM-ARY-L2H1-Fc4 1mg/kg SC | 72737.73 | 23.182 | 24 | 1294.342 |
| GM-ARY-L2H2-Fc4 1mg/kg SC | 49740.28 | 23.379 | 24 | 784.77 |

### Example 4. Pharmacokinetic Detection of Fusion Proteins Using Fc Region Containing YTE Mutation

The *in vivo* pharmacokinetics of fusion proteins GM-RY-L2H1-Fc4m and GM-ARY-L2H1-Fc4m of the present application were detected using cynomolgus monkeys. In the experiment, each group comprised 2 cynomolgus monkeys (half female, half male), and the cynomolgus monkeys in each group were subjected to a single administration of a corresponding test sample (1 mg/kg) via subcutaneous injection, and DULA (dulaglutide with no YTE mutation in the Fc region of IgG4) and DULA-Fc4m (dulaglutide with YTE mutation in the Fc region of IgG4) were used as controls. Blood samples were collected before administration and 4 h, 8 h, 24 h, 48 h, 72 h, 120 h, 168 h, 264 h, 336 h, 504 h, 672 h, 840 h, and 1008 h after administration. Serum was isolated, and the concentration of the test samples in the serum of the cynomolgus monkeys after administration of the test samples was measured by ELISA. The pharmacokinetic parameters were calculated using DAS (3.2.8) software. The results are shown in FIG. 3, FIG. 4, Table 4, and Table 5, where the data in Table 4 are from the curves of FIG. 3 and the data in Table 5 are from the curves of FIG. 4. Compared with dulaglutide also containing the YTE mutation, the GM-RY-L2H1-Fc4m (FIG. 3 and FIG. 4) and GM-ARY-L2H1-Fc4m (FIG. 4) had better *in vivo* half-life and exposure; the effect of dulaglutide with YTE mutation in Fc region was weak, and the YTE mutation in Fc region did not increase the *in vivo* half-life and exposure of dulaglutide compared with unmutated dulaglutide (FIG. 4).

Also taking the data in FIG. 1 into consideration, the half-life or exposure of the fusion proteins of the present application without YTE mutation in the Fc region was comparable to that of dulaglutide, and the half-life and exposure of the fusion proteins of the present application with YTE mutation in the Fc region were significantly higher than those of dulaglutide. This shows that YTE mutation of the Fc region significantly improves the pharmacokinetic properties of the fusion proteins of the present application, and the amino acid mutations of the human GLP-1 polypeptide variant of the fusion proteins and the YTE mutation of the Fc region have synergistic effect on the improvement of the pharmacokinetic properties.

**Table 4. Results of pharmacokinetic detection**

| Parameter | AUC(0-t) | t1/2 | Tmax | Cmax |
|---|---|---|---|---|
| | ug/L*h | h | h | ug/L |
| DULA-Fc4m 1mg/kg SC | 172710.369 | 21.379 | 6 | 5287.753 |
| GM-RY-L2H1-Fc4m 1mg/kg SC | 334876.633 | 53.8925 | 24 | 4152.8175 |

**Table 5. Results of pharmacokinetic detection**

| Parameter | AUC(0-t) | t1/2 | Tmax | Cmax |
|---|---|---|---|---|
| | ug/L*h | h | h | ug/L |
| DULA 1mg/kg SC | 167169.546 | 29.517 | 8 | 2688.837 |
| DULA-Fc4m 1mg/kg SC | 121353.801 | 28.9625 | 8 | 2956.3575 |
| GM-RY-L2H1-Fc4m 1mg/kg SC | 331986.252 | 82.9165 | 28 | 2867.4045 |
| GM-ARY-L2H1-Fc4m 1mg/kg SC | 379488.618 | 100.2185 | 8 | 3445.402 |

### Example 5. Pharmacokinetic Detection of Fusion Proteins Using Different Hinge Regions and Containing YTE Mutation in Fc Region

The *in vivo* pharmacokinetics of the fusion proteins GM-ARY-L2H2-Fc4m, GM-RY-L2H2-Fc4m, GM-ARY-L2H1-Fc4m, and GM-RY-L2H1-Fc4m of the present application containing YTE mutation in the Fc region were assayed according to the method in Example 4. The samples were taken for ELISA analysis. The pharmacokinetic parameters were calculated using DAS (3.2.8) software.

The results are shown in FIG. 5 and Table 6. The four fusion proteins all show good *in vivo* half-life or exposure, where the fusion protein using the hinge region of IgG1 (SEQ ID NO: 30) shows better overall effect.

**Table 6. Results of pharmacokinetic detection**

| Parameter | AUC(0-t) | t1/2 | Tmax | Cmax |
|---|---|---|---|---|
| | ug/L*h | h | h | ug/L |
| GM-ARY-L2H1-Fc4m 1mg/kg SC | 407554.621 | 50.352 | 8 | 4716.7835 |
| GM-RY-L2H1-Fc4m 1mg/kg SC | 271584.914 | 60.5895 | 16 | 3338.2635 |
| GM-ARY-L2H2-Fc4m 1mg/kg SC | 182545.608 | 38.795 | 16 | 2575.6805 |
| GM-RY-L2H2-Fc4m 1mg/kg SC | 188560.362 | 87.966 | 8 | 2870.56 |

### Example 6. Luciferase Detection for Activation of cAMP/PKA Signaling Pathway by Fusion Proteins of the Present Application

On the basis of the principle that fusion proteins of GLP-1 polypeptide variants bind to the GLP-1 receptor to activate adenylate cyclase and thereby promote an increase in the intracellular cyclic adenosine monophosphate (cAMP) level, the activation of the cAMP/PKA signaling pathway by the fusion proteins of the present application was detected using the CREB (cAMP response element binding protein) reporter gene. HER293 cells were transfected with the human GLP-1R plasmid and the CREB-driven luciferase reporter plasmid. After transfection, HER293-GLP1R-CREB-D4 cells were digested, harvested, and counted, and the cell density was adjusted to 4 × 10⁵ cells/mL in a DMEM medium containing 10% FBS. The cells were seeded into a 96-well plate at 50 µL/well. Test samples were each diluted with DMEM medium containing 10% FBS from 1000 ng/mL to obtain 9 concentrations; dulaglutide was used as a positive control. The test samples at various concentrations were then each added to the above 96-well plate at 50 µL/well, and the plate was incubated at 37 °C for several hours. The supernatant was discarded and luciferase substrate was added to each well. After the plate was left to stand at room temperature for 5 min, 50 µL of the mixture was taken and detected for luciferase activity on Bio-Glo^{™} Luciferase Assay System (Promega, G7940), and the plate was read using a microplate reader (Molecular Devices, SpectraMax M3). The fluorescence intensity reflects the level of cAMP and further the degree of GLP-1 receptor activation.

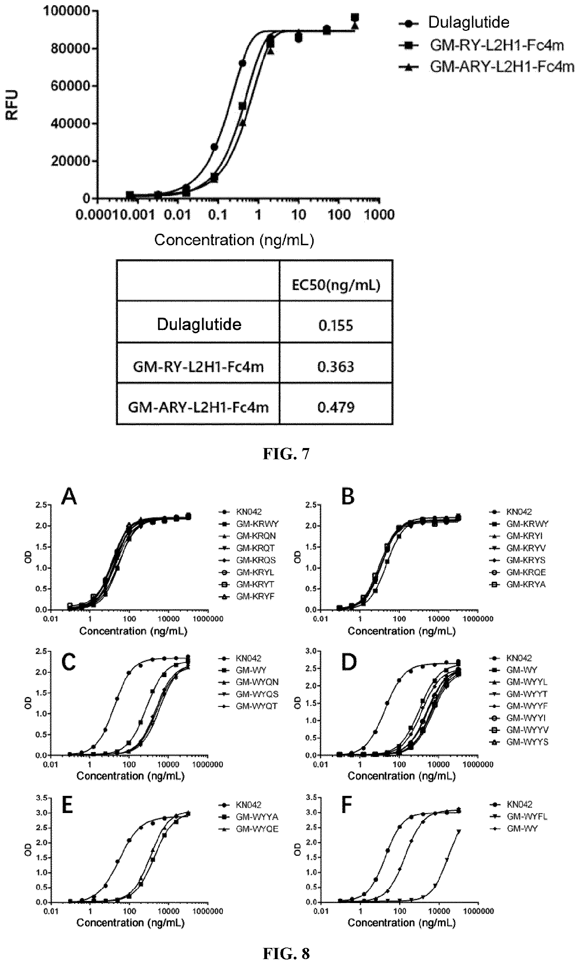

The results are shown in FIG. 7. The fusion proteins of the present application are comparable to dulaglutide in the ability to activate GLP-1 receptor.

### Example 7. Detection of GLP-1 Polypeptide Variants in Different Mutant Forms

The positive control dulaglutide used in the examples of the present application is a diabetes drug known in the art under the code number LY-2189265. Dulaglutide is referred to herein as GLP-1-Fc, DULA, or KN042.

To prepare a Fc fusion protein of the human GLP-1 polypeptide variant, a vector containing the Fc fusion protein nucleic acid sequence encoding the human GLP-1 polypeptide variant could be transfected into a cell, followed by expression and purification. The fusion protein comprises, in sequence from the N-terminus to the C-terminus, a GLP-1 polypeptide variant (the amino acid sequence is set forth in the table below), a linker peptide (G4S)₃, an Fc region derived from IgG4, and a hinge region. Sequences of exemplary human GLP-1 polypeptide variants are shown in the table below.

| Fusion protein | Human GLP-1 polypeptide variant | | |
|---|---|---|---|
| | Name | Amino acid mutation | SEQ ID NO |
| GM-WY | GLP-1-WY | W31Y | 71 |
| GM-KRWY | GLP-1-KRWY | W31Y, K26R | 4 |
| GM-KRQN | GLP-1-KRQN | K26R, Q23N | 72 |
| GM-KRQT | GLP-1-KRQT | K26R, Q23T | 73 |
| GM-KRQS | GLP-1-KRQS | K26R, Q23S | 74 |
| GM-KRQE | GLP-1-KRQE | K26R, Q23E | 75 |
| GM-KRYL | GLP-1-KRYL | K26R, Y19L | 5 |
| GM-KRYT | GLP-1-KRYT | K26R, Y19T | 6 |
| GM-KRYF | GLP-1-KRYF | K26R, Y19F | 7 |
| GM-KRYI | GLP-1-KRYI | K26R, Y19I | 8 |
| GM-KRYV | GLP-1-KRYV | K26R, Y19V | 9 |
| GM-KRYS | GLP-1-KRYS | K26R, Y19S | 10 |
| GM-KRYA | GLP-1-KRYA | K26R, Y19A | 11 |
| GM-WYQN | GLP-1-WYQN | W31Y, Q23N | 76 |
| GM-WYQS | GLP-1-WYQS | W31Y, Q23S | 77 |
| GM-WYQT | GLP-1-WYQT | W31Y, Q23T | 78 |
| GM-WYQE | GLP-1-WYQE | W31Y, Q23E | 79 |
| GM-WYYL | GLP-1-WYYL | W31Y, Y19L | 12 |
| GM-WYYT | GLP-1-WYYT | W31Y, Y19T | 13 |
| GM-WYYF | GLP-1-WYYF | W31Y, Y19F | 14 |
| GM-WYYI | GLP-1-WYYI | W31Y, Y19I | 15 |
| GM-WYYV | GLP-1-WYYV | W31Y, Y19V | 16 |
| GM-WYYS | GLP-1-WYYS | W31Y, Y19S | 17 |
| GM-WYYA | GLP-1-WYYA | W31Y, Y19A | 18 |
| GM-WYFL | GLP-1-WYFL | W31Y, F28L | 80 |
| GM-KRWYQN | GLP-1-KRWYQN | W31Y, K26R, Q23N | 81 |
| GM-KRWYQT | GLP-1-KRWYQT | W31Y, K26R, Q23T | 82 |
| GM-KRWYQS | GLP-1-KRWYQS | W31Y, K26R, Q23S | 83 |
| GM-KRWYQE | GLP-1-KRWYQE | W31Y, K26R, Q23E | 84 |
| GM-KRWYYL | GLP-1-KRWYYL | W31Y, K26R, Y19L | 20 |
| GM-KRWYYT | GLP-1-KRWYYT | W31Y, K26R, Y19T | 21 |
| GM-KRWYYI | GLP-1-KRWYYI | W31Y, K26R, Y19I | 22 |
| GM-KRWYYS | GLP-1-KRWYYS | W31Y, K26R, Y19S | 24 |
| GM-KRWYYA | GLP-1-KRWYYA | W31Y, K26R, Y19A | 25 |

### (1) Detection of binding affinity of fusion proteins for GLP-1 receptor

The binding affinity of the fusion proteins of Example 1 for the GLP-1 receptor was measured by ELISA. A fusion protein of the GLP-1 receptor and mouse Fc was expressed, and the fusion protein was referred to as GLP1R-muFc (SEQ ID NO: 3). GLP1R-muFc was diluted to 5 µg/mL and used to coat a 96-well plate; it was fixed at room temperature for 30 min. The plate was washed with PBS once, then blocked with 1% BSA at 37 °C for 30 min, and washed with 0.05% PBST20 three times. Test samples (4-fold serial dilutions, 10 µg/mL) were each added at 50 µL/well. KN042 was used as a positive control. The plate was incubated at 37 °C for 1 h and washed with PBST five times. Mouse monoclonal HP6023 anti-human IgG4Fc-HRP (abcam, ab99817, 2000-fold diluted with 0.05% PBST20 containing 1% BSA) was added at 50 µL/well. The plate was incubated at 37 °C for 1 h and washed with PBST five times. To each well was added 50 µL of TMB, and color development was allowed for 3 min. To each well was added 50 µL of 1 M H₂SO₄ to stop the reaction. The OD values were measured using a microplate reader (Molecular Devices, SpectraMax M3). KN042 was used as a positive control.

The results are shown in FIG. 8. Some of the fusion proteins containing double mutations exhibited good binding activity for the GLP-1 receptor.

### (2) Luciferase detection for activation of cAMP/PKA signaling pathway by fusion proteins

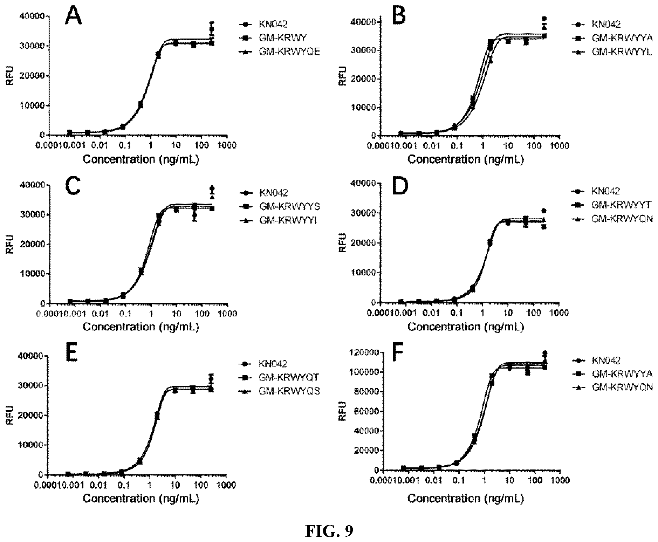

Reference was made to the method for luciferase detection for activation of cAMP/PKA signaling pathway by fusion proteins in Example 6 of the present application, and the results are shown in FIG. 9.

The results show that the fusion proteins claimed by the present application are all able to activate the GLP-1 receptor and thereby activate the cAMP/PKA signaling pathway.

### (3) Pharmacokinetic detection of fusion proteins

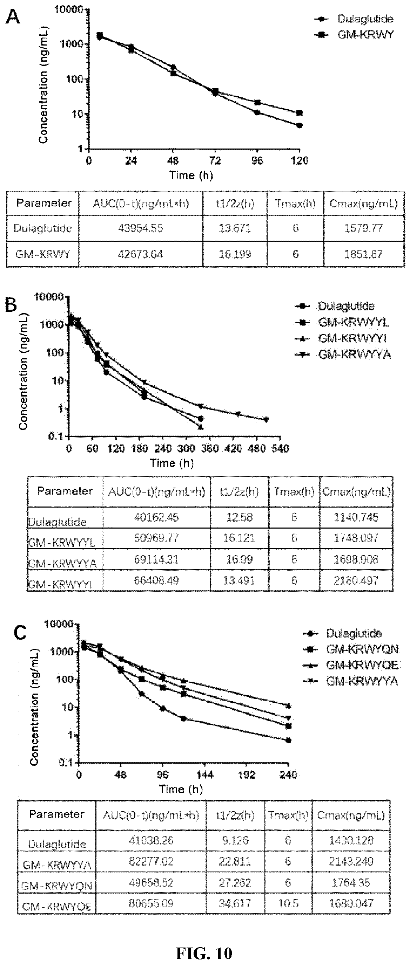

Reference was made to the method for pharmacokinetic detection of fusion proteins in Example 2 of the present application, and the results are shown in FIG. 10. FIG. 10A shows that the fusion protein GM-KRWY of the present application has a longer half-life (16.199 h) and higher drug exposure than dulaglutide and has a similar plasma concentration to dulaglutide. FIG. 10B shows that the fusion proteins GM-KRWYYL, GM-KRWYYA, and GM-KRWYYI of the present application have longer half-life (about 13 h to 16.5 h) than dulaglutide and have higher drug exposure and plasma concentration than dulaglutide. FIG. 10C shows that the fusion proteins GM-KRWYQN, GM-KRWYQE, and GM-KRWYYA of the present application have longer half-life (about 22 h to 25 h) than dulaglutide and have higher drug exposure and plasma concentration than dulaglutide.

### (4) Detection of oral glucose tolerance of fusion proteins

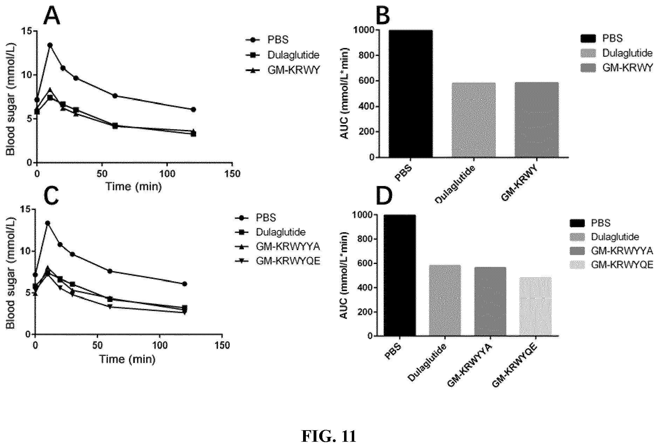

The *in vivo* hypoglycemic activity of the GLP-1-Fc fusion proteins was examined. Mice were fasted overnight and were randomly grouped after the blood sugar and body weight were measured prior to the experiment. Rats in each group were intraperitoneally injected with a corresponding test sample, and dulaglutide was used as a control. Each mouse was orally loaded with 1.5 g/kg glucose 0.5 h after administration. The mice were tested for tail-tip blood sugar level with a handheld Roche glucometer (Roche, Accu-chek) before glucose loading and 10 min, 20 min, 30 min, 60 min, and 120 min after loading. The mice's tail tips were cut off by about 1 mm, and the tails were squeezed from base to tip to collect blood from the veins. The blood was tested for blood sugar level. The results are shown in FIG. 11.

The results show that the fusion proteins GM-KRWY, GM-KRWYYA, and GM-KRWYQE of the present application have equivalent levels of *in vivo* hypoglycemic activity to the control.

The foregoing detailed description is provided by way of illustration and example and is not intended to limit the scope of the appended claims. Various variants of the embodiments currently listed in the present application will be apparent to those of ordinary skill in the art and are intended to be within the scope of the appended claims and equivalents thereof.

## Claims

1. A fusion protein, comprising a human GLP-1 polypeptide variant and an immunoglobulin Fc region;
wherein an amino acid sequence of the human GLP-1 polypeptide variant comprises at least 2 amino acid mutations compared with an amino acid sequence set forth in SEQ ID NO: 2, and the at least 2 amino acid mutations are located at amino acid positions selected from the group consisting ofW31, K26, and Y19.

2. The fusion protein according to claim 1, wherein the immunoglobulin Fc region is located at the C-terminus of the human GLP-1 polypeptide variant.

3. The fusion protein according to any one of claims 1-2, wherein the immunoglobulin Fc region is an Fc region derived from IgG.

4. The fusion protein according to any one of claims 1-3, wherein the immunoglobulin Fc region is an Fc region derived from IgG1, IgG2, IgG3, or IgG4.

5. The fusion protein according to any one of claims 1-4, wherein the immunoglobulin Fc region is an Fc region derived from IgG4.

6. The fusion protein according to any one of claims 1-5, wherein the immunoglobulin Fc region comprises an amino acid sequence set forth in any one of SEQ ID NOs: 32-35.

7. The fusion protein according to any one of claims 1-6, wherein the immunoglobulin Fc region comprises amino acid mutations, and the amino acid mutations selectively enhance binding affinity of the immunoglobulin Fc region for an Fc receptor compared with an immunoglobulin Fc region without amino acid mutations.

8. The fusion protein according to any one of claims 1-7, wherein the immunoglobulin Fc region comprises amino acid mutations at positions M252, S254, and T256 according to EU numbering.

9. The fusion protein according to claim 8, wherein the immunoglobulin Fc region comprises amino acid mutations in the group consisting of M252Y, S254T, and/or T256E.

10. The fusion protein according to claim 9, wherein amino acid mutations of the immunoglobulin Fc region are M252Y, S254T, and T256E.

11. The fusion protein according to any one of claims 7-10, wherein the immunoglobulin Fc region comprises an amino acid sequence set forth in SEQ ID NO: 36.

12. The fusion protein according to any one of claims 1-11, comprising a hinge region located between the human GLP-1 polypeptide variant and the immunoglobulin Fc region.

13. The fusion protein according to claim 12, wherein the hinge region is derived from IgG.

14. The fusion protein according to any one of claims 12-13, wherein the hinge region is derived from IgG1 or IgG4.

15. The fusion protein according to any one of claims 12-14, wherein the hinge region comprises an amino acid sequence set forth in any one of SEQ ID NOs: 30-31.

16. The fusion protein according to any one of claims 12-15, wherein the hinge region is derived from IgG1.

17. The fusion protein according to claim 16, wherein the hinge region comprises an amino acid sequence set forth in SEQ ID NO: 30.

18. The fusion protein according to any one of claims 1-17, further comprising a linker located between the human GLP-1 polypeptide variant and the hinge region.

19. The fusion protein according to claim 18, wherein the linker is a peptide linker.

20. The fusion protein according to any one of claims 18-19, wherein the linker has a length of 5-20 amino acids.

21. The fusion protein according to any one of claims 18-20, wherein the linker has a length of 15 amino acids.

22. The fusion protein according to any one of claims 18-21, wherein the linker comprises an amino acid sequence set forth in any one of SEQ ID NOs: 27-29.

23. The fusion protein according to any one of claims 18-22, wherein the linker comprises an amino acid sequence set forth in SEQ ID NO: 28.

24. The fusion protein according to any one of claims 1-23, wherein the human GLP-1 polypeptide variant has at least partial activity of human GLP-1.

25. The fusion protein according to any one of claims 1-24, wherein an amino acid sequence of the human GLP-1 polypeptide variant comprises mutations at amino acid positions W31 and K26 compared with the amino acid sequence set forth in SEQ ID NO: 2.

26. The fusion protein according to any one of claims 1-25, wherein an amino acid sequence of the human GLP-1 polypeptide variant comprises mutations at amino acid positions W31 and Y19 compared with the amino acid sequence set forth in SEQ ID NO: 2.

27. The fusion protein according to any one of claims 1-26, wherein an amino acid sequence of the human GLP-1 polypeptide variant comprises mutations at amino acid positions K26 and Y19 compared with the amino acid sequence set forth in SEQ ID NO: 2.

28. The fusion protein according to any one of claims 1-27, wherein an amino acid sequence of the human GLP-1 polypeptide variant comprises mutations at amino acid positions W31, K26, and Y19 compared with the amino acid sequence set forth in SEQ ID NO: 2.

29. The fusion protein according to any one of claims 1-28, wherein the human GLP-1 polypeptide variant comprises an amino acid substitution at the position W31, and the amino acid substitution is W31Y, W31R, W31K, or W31A.

30. The fusion protein according to claim 29, wherein the human GLP-1 polypeptide variant comprises an amino acid substitution at the position W31, and the amino acid substitution is W31Y

31. The fusion protein according to any one of claims 1-30, wherein the human GLP-1 polypeptide variant comprises an amino acid substitution at the position Y19, and the amino acid substitution is any amino acid substitution selected from the group consisting of Y19A, Y19L, Y19T, Y19F, Y19I, Y19Y, and Y19S.

32. The fusion protein according to claim 31, wherein the human GLP-1 polypeptide variant comprises an amino acid substitution at the position Y19, and the amino acid substitution is any amino acid substitution selected from the group consisting of Y19A.

33. The fusion protein according to any one of claims 1-32, wherein the human GLP-1 polypeptide variant comprises an amino acid substitution at the position K26, and the amino acid substitution is K26R.

34. The fusion protein according to any one of claims 1-33, wherein the human GLP-1 polypeptide variant comprises an amino acid sequence set forth in SEQ ID NO: 26.

35. The fusion protein according to any one of claims 1-34, wherein the human GLP-1 polypeptide variant comprises an amino acid sequence set forth in any one of SEQ ID NOs: 4-25.

36. The fusion protein according to any one of claims 1-35, wherein an amino acid sequence of the human GLP-1 polypeptide variant comprises amino acid mutations W31Y, K26R, and Y19A compared with the amino acid sequence set forth in SEQ ID NO: 2.

37. The fusion protein according to claim 36, wherein the human GLP-1 polypeptide variant comprises an amino acid sequence set forth in SEQ ID NO: 25.

38. The fusion protein according to any one of claims 1-35, wherein an amino acid sequence of the human GLP-1 polypeptide variant comprises amino acid mutations W31Y and K26R compared with the amino acid sequence set forth in SEQ ID NO: 2.

39. The fusion protein according to claim 38, wherein the human GLP-1 polypeptide variant comprises an amino acid sequence set forth in SEQ ID NO: 4.

40. The fusion protein according to any one of claims 1-39, comprising the human GLP-1 polypeptide variant and the immunoglobulin Fc region.

41. The fusion protein according to any one of claims 12-40, comprising the human GLP-1 polypeptide variant and the hinge region.

42. The fusion protein according to any one of claims 12-41, comprising the human GLP-1 polypeptide variant, the immunoglobulin Fc region, and the hinge region.

43. The fusion protein according to any one of claims 18-42, comprising, in sequence from the N-terminus to the C-terminus, the human GLP-1 polypeptide variant, the linker, the hinge region, and the immunoglobulin Fc region.

44. The fusion protein according to any one of claims 1-43, comprising an amino acid sequence set forth in any one of SEQ ID NOs: 37-45.

45. The fusion protein according to any one of claims 1-44, comprising an amino acid sequence set forth in any one of SEQ ID NOs: 43-44.

46. An isolated nucleic acid molecule encoding the fusion protein according to any one of claims 1-45.

47. A vector, comprising the isolated nucleic acid molecule according to claim 46.

48. A cell, comprising or expressing the fusion protein according to any one of claims 1-45, the isolated nucleic acid molecule according to claim 46, or the vector according to claim 47.

49. A pharmaceutical composition, comprising the fusion protein according to claims 1-45, the isolated nucleic acid molecule according to claim 46, the vector according to claim 47, and/or the cell according to claim 48, and optionally a pharmaceutically acceptable carrier.

50. An immunoconjugate, comprising the fusion protein according to any one of claims 1-45.

51. Use of the fusion protein according to any one of claims 1-45, the isolated nucleic acid molecule according to claim 46, the vector according to claim 47, the cell according to claim 48, the pharmaceutical composition according to claim 49, and/or the immunoconjugate according to claim 50 in preparing a medicament for preventing and/or treating a metabolic disease or condition.

52. The use according to claim 51, wherein the metabolic disease or condition comprises a GLP-1-associated metabolic condition.

53. The use according to any one of claims 51-52, wherein the metabolic disease or condition comprises diabetes.

54. A method for preventing and/or treating a metabolic disease or condition, comprising administering to a subject in need thereof the fusion protein according to any one of claims 1-45, the isolated nucleic acid molecule according to claim 46, the vector according to claim 47, the cell according to claim 48, the pharmaceutical composition according to claim 49, and/or the immunoconjugate according to claim 50.

55. The method according to claim 54, wherein the metabolic disease or condition comprises a GLP-1-associated metabolic condition.

56. The method according to any one of claims 54-55, wherein the metabolic disease or condition comprises diabetes.

57. The fusion protein according to any one of claims 1-45, the isolated nucleic acid molecule according to claim 46, the vector according to claim 47, the cell according to claim 48, the pharmaceutical composition according to claim 49, and/or the immunoconjugate according to claim 50 for use in preventing and/or treating a metabolic disease or condition.

58. The fusion protein, the isolated nucleic acid molecule, the vector, the cell, and/or the pharmaceutical composition according to claim 57, wherein the metabolic disease or condition comprises a GLP-1-associated metabolic condition.

59. The fusion protein, the isolated nucleic acid molecule, the vector, the cell, and/or the pharmaceutical composition according to any one of claims 57-58, wherein the metabolic disease or condition comprises diabetes.

60. A method for increasing or promoting insulin expression in a subject in need thereof, comprising administering to the subject an effective amount of the fusion protein according to any one of claims 1-45, the isolated nucleic acid molecule according to claim 46, the vector according to claim 47, the cell according to claim 48, the pharmaceutical composition according to claim 49, and/or the immunoconjugate according to claim 50.
